# EUROPEAN PATENT APPLICATION

(11) **EP 0 771 545 A1**
(43) Date of publication of application: **07.05.1997**
(21) Application number: 96202960.9
(22) Date of filing: 23.10.1996
(51) Int. Cl.: A61B 1/31

(54) **Disposable rectoscope**

(30) Priority: 02.11.1995 IT MI952266
(71) Applicant: Sapimed S.r.l., 15100 Alessandria (IT)
(72) Inventor: Oddenino, Gian Paolo, 15100 Alessandria (IT)
(74) Representative: De Nova, Roberto

(57) **Abstract**

A disposable rectoscope (1), which achieves enhanced rectal examination and at the same time has structural simplicity, comprises a handle (3) and an examination tube (2) formed by a first portion (4) of greater length and a second portion (5) of short length, the second portion (5) being located in extension of the first portion (4) and being connected to the first portion (4) in an orientable manner under the action of means (6) of control.

## Description

The present invention relates to a disposable rectoscope of the type comprising an examination tube and a handle.

Known disposable rectoscopes make it possible for the doctor to carry out a precise examination of the rectal canal within its essentially rectilinear part.

However, these do not make it possible to carry the examination beyond the point, known as the sigmoid flexure, at which the rectal canal changes direction. In this case, it is in fact necessary to turn to specialist doctors who have appropriate professional equipment which is well-known for its cost and complexity.

The problem which forms the basis of the present invention is that of devising a disposable rectoscope which has structural and functional characteristics such as to overcome the abovementioned disadvantage.

This aim is achieved by a disposable rectoscope of the type specified, which is characterized in that the examination tube comprises a first portion of greater length, a second portion of short length located in extension of the first portion and connected to the first portion in an orientable manner, and also means of control for controlling the orientation of the second portion in relation to the first.

Further characteristics and the advantages of the rectoscope according to the present invention will emerge from the following description which relates to an exemplary embodiment thereof and is given by way of non-limiting example with reference to the attached drawings, in which:
- Figure 1 shows a lateral view in section of a rectoscope according to the invention, taken along the line I-I,
- Figure 2 shows a rear view of the rectoscope in Figure 1, taken on the arrow II,
- Figure 3 shows a plan view of a detail of the rectoscope in Figure 1, taken on the arrow III,

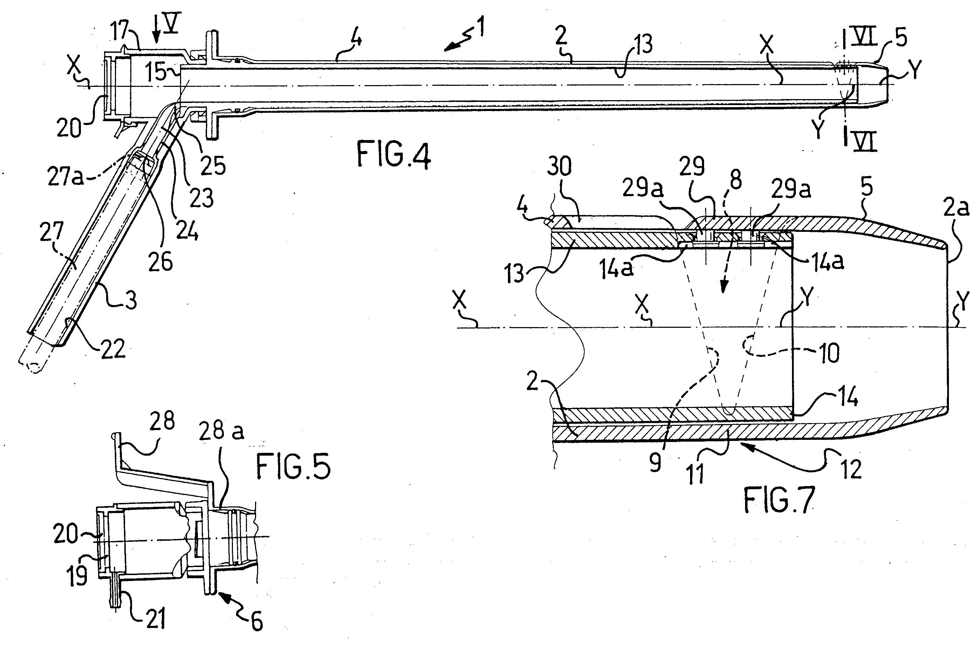

- Figure 4 shows a lateral view in section of the rectoscope in Figure 1, at a different stage in its functioning,
- Figure 5 shows a plan view of a detail of the rectoscope in Figure 4, taken on the arrow V,
- Figure 6 shows a view in section and on enlarged scale of a detail of the rectoscope in Figure 4, taken along the line VI-VI,
- Figure 7 shows a view in section and on enlarged scale of a detail of the rectoscope in Figure 4, taken along the line VII-VII,

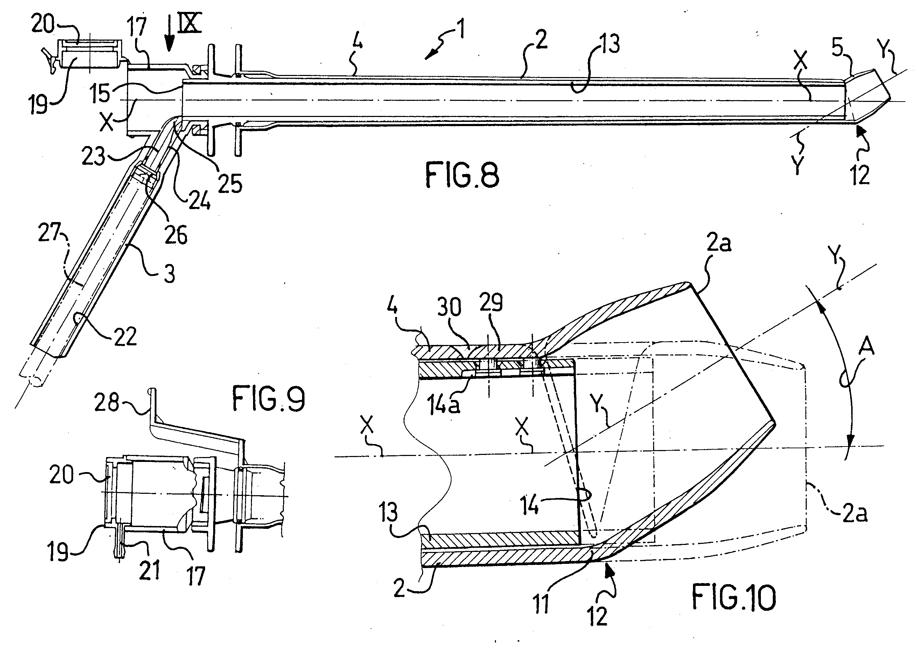

- Figure 8 shows a lateral view in section of the rectoscope in Figure 1 at another stage in its functioning,
- Figure 9 shows a plan view of a detail of the rectoscope in Figure 8 taken on the arrow IX, and
- Figure 10 shows a view in section and on enlarged scale of a detail of the rectoscope in Figure 8.

With reference to the attached figures, 1 indicates globally a disposable rectoscope for rectal examination.

The rectoscope 1 comprises an examination tube 2 which has a free end 2a, and a handle 3.

The examination tube 2, in accordance with the invention, comprises a first portion 4 of greater length having an axis x-x, and a second portion 5 of short length having an axis y-y.

The second portion 5 is located in extension of the first portion 4 and is connected to the first portion 5 in an orientable manner, so that its axis y-y forms an angle A with the axis x-x which is selected within a range extending between A = 0 and A = 30°.

The rectoscope 1 also comprises means of control 6 which are provided for controlling the orientation of the second portion 5 in relation to the first portion 4.

The first portion 4 and the second portion 5 of the examination tube 2 are made in a single piece, indicated by 7, which is obtained by moulding an appropriate elastically deformable plastic such as polyethylene. In said single piece 7, of circular section, a V-shaped notch 8 is formed, which pertains to the greater part of the section of the piece 7 and defines, with the opposite sides of the V, opposite facing ends of the two portions 4 and 5, indicated respectively by 9 and 10. The notch 8 defines, in the region of the vertex of the V, a deformable bridge 11 which constitutes a hinge 12, around which the second portion 5 of the examination tube can be oriented in relation to the first portion 4.

The means of control 6 for controlling the orientation of the second portion 5 in relation to the first portion 4 comprise a tubular pipe 13 which is obtained by moulding an appropriate transparent plastic material, for example polystyrene. The pipe 13 extends coaxially with the first portion 4, is guided slidably therein and has opposite ends 14 and 15 projecting therefrom.

In particular, the end 14 is fitted into the second portion 5 and is fixed to the end 10 of the second portion 5 in a position diametrically opposite the hinge 12. The opposite end 15 is snap-coupled in a sealed manner to one end 16 of a sleeve 17 which extends coaxially with the axis x-x.

The sleeve 17 has an opposite end 18 provided with a small door 19 which is hinged on the sleeve by means of a deformable bridge 19a and can be moved between an open position and a sealed snap-closed position on the sleeve. The small door 19 is provided with a window 20 which allows viewing through the sleeve and the examination tube when the small door is in the closed position.

Leading to the small door 19 is a connection 21 for a small tube for insufflation of air.

Integral with the sleeve 17 is the handle 3 which is intended to be grasped with, for example, the right hand.

The handle 3 is hollow, having a seat 22 for receiving a light-guide insert 23 made of a transparent plastic material, for example polystyrene.

The light-guide insert 23 is surrounded by a bush 24 made of opaque material and has a first, curved end 25 which is in contact with the end 15 of the pipe 13, and a second end 26 which is intended to come into contact with the terminal 27a of an optical fibre 27 connected to a cold-light source.

The first portion 4, in the region of an end 28a opposite the end 9, is equipped with a hand-grip 28 which is intended to be grasped with, for example, the left hand.

As far as the fixing of the pipe 13 to the second portion 5 is concerned, the end 14 of the pipe is fixed to a projection 29 jutting out from the second portion 5. A recess 30, which mates with the projection 29, is formed in the first portion 4, so as to receive the projection 29 when the angle A between the second portion 5 and the first portion 4 has the maximum value of 30°.

Two mushroom-shaped protuberances 29a project from the projection 29 and engage in the manner of press-buttons two corresponding seats 14a formed in the region of the end 14 of the pipe 13.

The rectoscope 1 is completed by a mandrel 31 which is intended to be inserted through the sleeve 17, with the small door 19 open, through the pipe and the examination tube, with the portion 5 aligned with the portion 4, so as to project outside the free end 2a and thus to favour the insertion of the examination tube of the rectoscope into the rectal canal of the patient.

The mandrel 31 comprises a first portion 32 with a cross-shaped section and an ogive-shaped end portion 33 which are interconnected by a bridge 34 constituting a hinge.

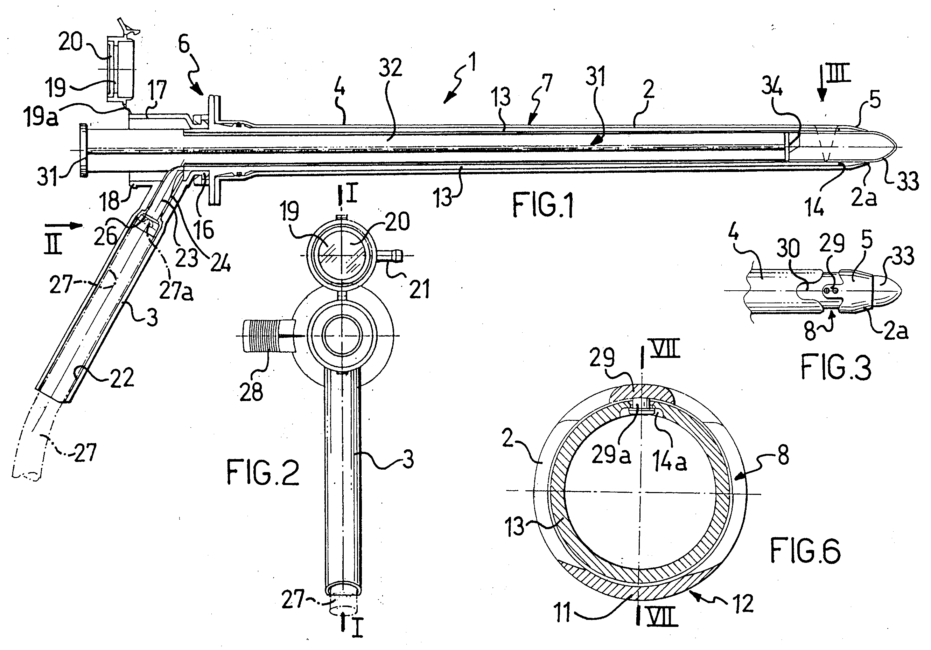

In operation, the doctor inserts into the rectal canal of the patient the examination tube of the rectoscope with the first and second portion in alignment (Fig. 1) until the free end 2a arrives at the deflection of the rectal canal. In this state, the doctor, by acting with the right hand and the left hand, respectively, on the handle and on the hand-grip, brings about mutual sliding between the first portion and the pipe, and consequently orients the second portion, causing it to adopt an appropriate inclination A in such a manner that the second portion assumes the orientation of the rectal canal beyond the deflection. At this point, the examination tube can be further inserted.

When this has been done, the doctor performs the opposite manoeuvre, bringing about mutual sliding in the opposite direction and therefore aligning the second portion with the first portion again. In this way, the part of the rectal canal beyond the deflection is straightened and rendered examinable.

By advancing in this manner repeatedly, successive parts of the rectal canal are made accessible for examination.

The main advantage of the rectoscope according to the invention lies in the enhanced capacity for examination of the rectal canal. The field of diagnosis is thus made very much broader and the doctor acquires operational capabilities which were previously available only to specialist doctors equipped with costly and complex professional equipment.

A further advantage of the rectoscope according to the present invention lies in its structural simplicity, so that it lends itself to mass production for disposable use.

Clearly, an expert in the field can, for the purpose of satisfying contingent and specific requirements, subject the rectoscope described above to numerous modifications and variations, all of which are, however, within the scope of protection of the invention, as defined by the following claims.

## Claims

1. Disposable rectoscope (1) of the type comprising an examination tube (2) and a handle (3), characterized in that the examination tube (2) comprises a first portion (4) of greater length, a second portion (5) of short length located in extension of the first portion (4) and connected to the first portion (4) in an orientable manner, and also means (6) of control for controlling the orientation of the second portion (5) in relation to the first portion (4).

2. Disposable rectoscope (1) according to Claim 1, characterized in that the first portion (4) and the second portion (5) are formed by a single piece (7) made of a plastic material, and in that, in said single piece (7), a V-shaped notch (8) is formed, which defines, with the sides of the V, opposite facing ends (9, 10) of the two portions (4, 5), and, with the vertex of the V, a deformable bridge (11) which constitutes a hinge (12) around which the second portion (5) can be oriented in relation to the first portion (4).

3. Disposable rectoscope (1) according to Claim 2, characterized in that the means (6) of control comprise a pipe (13) which extends coaxially along said single piece (7), is guided slidably along the first portion (4) and has one end (14) fixed to the second portion (5).

4. Disposable rectoscope (1) according to Claim 3, characterized in that said end (14) of the pipe (13) is fixed to a projection (29) jutting out from the second portion (5), and in that a recess (30) which mates with said projection (29) is formed in the first portion (4).

5. Disposable rectoscope (1) according to Claim 4, characterized in that said end (14) of the pipe (13) and the projection (29) are fixed by means of at least one mushroom-shaped protuberance (29a) formed in the projection (29), in engagement in the manner of a press-button in a corresponding seat (14a) formed in the pipe (13).

6. Disposable rectoscope (1) according to Claim 5, characterized in that the pipe (13) comprises an opposite end (15) associated with the handle (3) and in that the first portion (4) of the examination tube (2) is equipped with a hand-grip (28).

7. Disposable rectoscope (1) according to Claim 6, characterized in that the pipe (13) is made of a transparent plastic material, and in that said opposite end (15) is faced by a light-guide insert (23) which is made of transparent plastic material, accommodated in a seat (22) formed in the handle (3), and intended to be put in contact with a terminal (27a) of an optical fibre (27) connected to a cold-light source.
